# EUROPÄISCHE PATENTSCHRIFT

(11) **EP 2 931 378 B1**
(45) Veröffentlichungstag und Bekanntmachung des Hinweises auf die Patenterteilung: **27.02.2019**
(21) Anmeldenummer: 13799077.6
(22) Anmeldetag: 04.12.2013
(51) Int. Cl.: A61Q 5/00, A61Q 19/00, A61K 8/81, C08F 2/22, C08F 2/26, C08F 220/18, C08F 220/26

(54) **KOSMETISCHE ZUBEREITUNGEN MIT FLIESSGRENZE**
COSMETIC PREPARATIONS WITH A FLOW POINT
PRÉPARATIONS COSMÉTIQUES À LIMITE RHÉOLOGIQUE

(30) Priorität: 12.12.2012 DE 102012222956
(43) Veröffentlichungstag der Anmeldung: 21.10.2015
(73) Patentinhaber: Beiersdorf AG, 20253 Hamburg (DE)
(72) Erfinder: ARGEMBEAUX, Horst, 21465 Wentorf (DE); KUMMER, Andreas, B., 21079 Hamburg (DE); COUNRADI, Katrin, 22143 Hamburg (DE); RASCHKE, Thomas, 25421 Pinneberg (DE)
(86) Internationale Anmeldenummer: PCT/EP2013/075495
(87) Internationale Veröffentlichungsnummer: WO 2014/090651

(56) Entgegenhaltungen:
- WO-A1-2011/151091
- WO-A1-2012/006402
- WO-A1-2012/168015
- WO-A1-2013/017328
- DE-A1- 19 909 803
- US-A1- 2003 207 988
- DATABASE GNPD [Online] MINTEL; 1. Juni 2013 (2013-06-01), "Quen of the Night Shower & Oil Pearls", XP002720605, Database accession no. 2099910
- Anonymous: "On Surfactants and Formulation (face wash, shampoo and shower gels) - It's all in my hands", , 23 April 2013 (2013-04-23), XP055301976, Retrieved from the Internet: URL:https://itsallinmyhands.com/2013/04/23 /on-surfactants-and-formulation-face-wash- shampoo-and-shower-gels/ [retrieved on 2016-09-13]
- Anonymous: "Poly(ethylene glycol) behenyl ether methacrylate solution average Mn ~1,500, 50 wt. % in methacrylic acid/water, contains 1000 ppm MEHQ as stabilizer, 25% water | Sigma-Aldrich", , 16 March 2016 (2016-03-16), XP055355499, Retrieved from the Internet: URL:http://www.sigmaaldrich.com/catalog/pr oduct/aldrich/468258?lang=de ion=DE [retrieved on 2017-03-16]

## Beschreibung

Die vorliegende Erfindung beschreibt transparente wässrige, tensidhaltige Zubereitungen, die ein oder mehrere vernetzte Acrylat Copolymere enthalten. Derartige Zubereitungen können auch suspendierte oder stabilisierte Partikel aufweisen.

Bisher ist es nicht möglich gewesen, transparente Tensid-Formulierungen mit stabilisierten Partikeln bei einem pH-Wert von 4,0 bis 7,0, insbesondere pH-Wert < 6,4 bereitzustellen, die geeignete Fließeigenschaften aufweisen, mit einer Viskosität ≥ 2000mPa·s ausgestattet sind und ein hohes Schaumvermögen aufweisen. Zudem weisen bekannte Rezepturen einen anionischen Tensidanteil von >10% auf. Durch den hohen Anteil von anionischen Tensiden haben Sie eine schlechte Milde. Es ist nun insbesondere wünschenswert, Zubereitungen zur Verfügung zu stellen, die neben der Transparenz, den geeigneten Fließeigenschaften, die eine stabile Suspendierung von Partikeln, Tröpfchen und/oder Bläschen ermöglichen und einem weitgehend sauren pH-Wert, kosmetisch vorteilhafte Zubereitungen sind, die sich durch eine gute Milde und pflegende Eigenschaften auszeichnen.

Der Stand der Technik kennt zwar bereits Systeme mit Xanthangummi. Diese besitzen aber bezüglich des Hautgefühls während und nach der Anwendung kosmetische Eigenschaften, die verbessert werden können. Darüber hinaus werden bei gleicher Einsatzkonzentration nur geringere Viskositätswerte erreicht.

Um Fortschritte im Bereich der Stabilisierung gelartiger Systeme bei gleichzeitiger Verbesserung der Anwendungseigenschaften zu erzielen, wurden Versuche unternommen bei denen Xanthangummi durch polymere Strukturen ersetzt wurde. Besonders geeignet erwiesen sich vernetzte acrylat-basierte Polymere. Durch den Einsatz dieser polymeren Strukturanten konnten Zubereitungen zur Verfügung gestellt werden, die eine Fließgrenze ausbilden. Dadurch gelingt es feste, Partikel, Tröpfchen und Blasen in wässrigen Systemen zu stabilisieren. Diese so erhaltenen Zubereitungen weisen meistens einen pH-Wert ≥ 5,5 auf. Bei Zubereitungen des Standes der Technik, die auch unterhalb pH 5,5 zu transparenten Zubereitungen mit Fließgrenze führen, treten bei längerer Lagerung Eintrübungen auf und die kosmetische Konsistenz ist unvorteilhaft und bedarf der Verbesserung.

Bei der Herstellung von acrylatbasierten Polymeren spielen die Auswahl der Monomeren, das Verhältnis der Monomeren untereinander und der Ablauf der Polymerisationsreaktion eine wichtige Rolle. Besonders der Ablauf der Reaktion hat Einfluss auf die Eigenschaften der entstehenden Produkte. Die Polymerisationsreaktion kann folgendermaßen durchgeführt werden: Zunächst versetzt man Monomere mit einem Initiator (beispielsweise Ammoniumperoxodisulfat) um die Reaktion zu starten. Nach 30 Minuten erfolgt eine Zugabe von Vernetzermolekülen und eine weitere Monomerzugabe. Die Reaktion verläuft dann für 4 Stunden. Variationen in den Reaktionszeiten sind möglich. Die entstehenden Produkte weisen einen homogenen Aufbau auf. Die Monomere, die eingesetzt werden, sind beispielsweise:
- saure Vinylmonomere und/oder ihre Salze,
- nichtionische Vinylmonomere, bevorzugt hydrophobe nichtionische Monomere,
- Monomere, die eine ungesättigte Endgruppe sowie einen Polyoxyalkylenteil enthalten und
- quervernetzende Monomere.

Beispielhaft wird im Folgenden die Herstellung von homogenen quervernetzten acrylatbasierten Coplymeren beschrieben. Diese Polymere sind erhältlich durch radikalische Emulsionspolymerisation von (A) wenigstens einem sauren Vinylmonomer oder dessen Salz, (B) wenigstens einem nichtionischen Vinylmonomer, besonders bevorzugt einem hydrophoben nichtionischen Vinylmonomer, (C) wenigstens einem Monomer, enthaltend eine ungesättigte Endgruppe und einen Polyoxyalkylenteil, (D) wenigstens einem quervernetzenden Monomeren, (E) optional einem Schutzkolloid dadurch gekennzeichnet, dass die Polymerisation so gesteuert wird, dass (F) zumindest zeitweise der Geleffekt auftritt, dadurch erreicht, dass die Monomerzugabe der Monomere (A), (B) und (C) (Dosierzeit) während 120 Minuten, bevorzugt 60 Minuten, besonders bevorzugt 40 Minuten, ganz besonders bevorzugt 30 Minuten erfolgt und (G) die Zugabe des quervernetzenden Monomeren (D) frühestens 10 Minuten, bevorzugt frühestens 15 Minuten nach der ersten Zugabe der Monomere (A), (B) und (C) beginnt. Bei einer solchen Polymerisation unter Ausnutzung des Trommsdorff-Effektes, d.h. bei konstanter Zugabe der Monomere und gleichzeitig hoher Zugabegeschwindigkeit der Monomere bildet sich ein Monomerenüberschuß, der zu einer Selbstbeschleunigung der Polymerisation (Trommsdorff-Effekt) führt. Das Ergebnis ist ein Anstieg der Molekulargewichte bei gleichzeitig vorteilhafter Morphologie der Polymere. Dabei ist es von Vorteil, wenn die Temperatur während der Polymerisation zwischen 70 und 90°C, bevorzugt 80 und 90°C gehalten wird. Weiter von Vorteil ist es, wenn die Initiatorzugabe sowohl vor Beginn der Dosierzeit, als auch nach Zugabe des quervernetzenden Monomers erfolgt. Dabei ist es besonders bevorzugt, wenn (H) assoziative Monomere fehlen oder höchstens eine Konzentration von 1 Gew.%, bevorzugt 0,1 Gew.% aufweisen. **D**as saure Vinylmonomer (A) wird gewählt unter Vinylmonomeren ausgewählt aus Acrylsäure oder Methacrylsäure oder deren Alkali-, Erdalkali-, Ammonium oder Alkylammoniumsalzen. Das nichtionische Vinylmonomer (B) wird gewählt aus der Gruppe der C1-C22-Alkylacrylate und der C1-C22-Alkylmethacrylate sowie deren Mischungen. Dadurch werden gute Fließeigenschaften und damit ein vorteilhaftes rheologisches Profil erreicht. Das Monomer (C), enthaltend eine ungesättigte Endgruppe und einen Polyoxyalkylenteil wird gewählt unter Allylpolyethylenglykolether mit 30 Ethylenoxyeinheiten, Allylpolyethylenglykolether mit 20 Ethylenoxyeinheiten und CH2=CHCH2O[(CH2CH2O)n(CH2(CH3)CHO)]mCH3 mit m+n = 5 bis 100 und n/m = 1, bevorzugt werden eingesetzt Emulsogen RAL307 (Allylpolyalkylenglykolether (EO 30 mol), Clariant), Polyglykol A11/1800 (Allylpolyalkylenglykolether (EO 20 mol, PO 20 mol), Clariant), Pluriol A111R (Allylalkoholalkoxylate, BASF) oder Polyglykol AB25-8 (Polyalkylenglykolallylbutylether (EO 25 mol, PO 8 mol), Clariant). Besonders bewährt hat sich Polyglykol A11/1800, was daher besonders bevorzugt ist. Das quervernetzende Monomer (D) wird gewählt unter Polyol(meth)acrylaten mit wenigstens zwei (Meth)acrylatgruppen und den Mischestern von Polyolen mit Acrylsäure und/oder Methacrylsäure. Weiter besonders bevorzugt ist es, wenn die Monomere (A) in Gehalten von 10 bis 75%, bevorzugt 20 bis 60%, besonders bevorzugt 28 bis 52%, ganz besonders bevorzugt 32 bis 52%, (B) in Gehalten von 10 bis 90%, bevorzugt 30 bis 80%, besonders bevorzugt 40 bis 62%, ganz besonders bevorzugt 40 bis 60%, (C) in Gehalten von 0,5 bis 40%, bevorzugt 1 bis 10%, besonders bevorzugt 2 bis 6%, (D) in Gehalten von bis zu 1%, bevorzugt 0,05 bis 0,5%, besonders bevorzugt 0,1 bis 0,3% vorliegen. Ganz besonders bevorzugt ist es, wenn die Monomere (A):(B) in Massenverhältnissen von 1:2,2 bis 1,5:1, bevorzugt 1:1,6 bis 1,3:1 vorliegen. Ganz besonders bevorzugt ist es, wenn die Zugabe des quervernetzenden Monomeren frühestens nach 10 Minuten, besonders bevorzugt nach 15 Minuten beginnt und entweder sofort endet oder bis zum Ende der Dosierzeit der Monomere andauert.

Die Produkte, die durch derartige Reaktionen entstehen, kann man als homogene, quervernetzte, acrylatbasierte Copolymere bezeichnen. In der DE 102011078087 werden beispielsweise derartige Reaktionen und Produkte beschrieben. Homogene, quervernetzte, acrylatbasierte Copolymere, die mit der in der DE 102011078087 beschriebenen Herstellmethode gewonnen werden, werden im Folgenden als AMA-X-Polymere bezeichnet. Die Lehre zur Herstellung der oben aufgeführten AMA-X-Polymere wurde in DE 102011078087 offenbart. DE 102011078087 wird hiermit in seiner Gesamtheit in die Offenbarung dieser Anmeldung eingeschlossen.

Es hat sich nun für den Fachmann überraschend herausgestellt, dass wässrige, tensidhaltige Zubereitungen enthaltend ein oder mehrere AMA-X-Polymere den Nachteilen des Standes der Technik abhelfen.

Erfindungsgemäß bevorzugt ist es, wenn die Konzentration von wenigstens einem AMA-X-Polymer 0,1 bis 5,0 %, bevorzugt 0,5 bis 4,0 %, besonders bevorzugt 1,0 bis 2,0 %, bezogen auf den Aktivgehalt und das Gesamtgewicht der Zubereitung, beträgt.

Es ist auch erfindungsgemäß, wenn die erfindungsgemäßen wässrigen, tensidhaltigen Zubereitungen Partikel, Tröpfchen und/oder Bläschen enthalten, die weitgehend stabil suspendiert sind und auch weitgehend stabil suspendiert bleiben.

Es ist ebenfalls erfindungsgemäß, wenn die erfindungsgemäßen wässrigen, tensidhaltigen Zubereitungen mit oder ohne Partikel, Tröpfchen und/oder Bläschen transparente Zubereitungen sind, wobei transparente Zubereitungen durch Trübungswerte < 30 NTU, bevorzugt < 25 NTU, besonders bevorzugt ≤ 20 NTU charakterisiert sind.

Erfindungsgemäß ist ebenfalls, dass die oben beschriebenen Zubereitungen einen pH-Wert von 4,0 bis 7,0 aufweisen, bevorzugt von 4,0 bis < 6,4, besonders bevorzugt von 4,0 bis ≤ 5,5.

Es ist erfindungsgemäß besonders bevorzugt, wenn die erfindungsgemäßen Zubereitungen einen pH-Wert von 4,0 bis 7,0 aufweisen, bevorzugt von 4,0 bis < 6,4, besonders bevorzugt von 4,0 bis ≤ 5,5 und transparente Zubereitungen sind, charakterisiert durch Trübungswerte < 30 NTU, bevorzugt <25 NTU, besonders bevorzugt ≤ 20 NTU.

Erfindungsgemäß bevorzugt ist auch, dass die erfindungsgemäßen Zubereitungen eine Viskosität ≥ 2000mPa·s aufweisen.

Erfindungsgemäß ist auch, dass die tan delta-Werte für die oben beschriebenen Zubereitungen bei 0,05 bis 0,6, bevorzugt bei 0,1 bis 0,5 liegen.

Erfindungsgemäß bevorzugt ist es auch, dass die oben beschriebenen Zubereitungen eine Fließgrenze von 0,5 bis 20 Pa, bevorzugt 1 bis 6 Pa aufweisen. Weiterhin ist erfindungsgemäß dass Partikel, Tröpfchen und/oder Blasen nahezu homogen in der Zubereitung verteilt sind und auch bleiben. Die Partikel oder Tröpfchen weisen einen Durchmesser von 0,1 bis 2000 µm auf, weiterhin eine Dichte von 0,001 bis 2 g/cm³.

Auch erfindungsgemäß bevorzugt ist, dass die erfindungsgemäßen Zubereitungen, insbesondere transparente Reinigungszubereitungen frei von Xanthangummi sind. Weiterhin erfindungsgemäß ist, dass die Zubereitungen eine Viskosität ≥ 2000mPa·s haben und frei von Xanthangummi sind.

Erfindungsgemäß bevorzugt ist es ebenfalls, wenn neben AMA-X-Polymeren weitere Struktur-bildende Polymere enthalten sind. Weiterhin erfindungsgemäß ist, wenn die Konzentration an AMA-X-Polymeren und weiteren Struktur-bildenden Polymeren 0,1 bis 5,0 Gew.-%, bevorzugt 0,2 bis 4,0 Gew.-%, besonders bevorzugt 0,5 bis 2,0 Gew.-%, bezogen auf den Aktivgehalt in den Zubereitungen, beträgt.

Erfindungsgemäß ist es ebenfalls, dass die Zubereitungen 1,0 bis 15 Gew.-% anionische Tenside enthalten, bevorzugt 1,0 bis < 12 Gew.-%.

Erfindungsgemäß ist es weiterhin, dass die anionischen Tenside in Kombination mit 1,0 bis 20 Gew.-% amphoteren, nichtionischen und/oder kationischen Tensiden eingesetzt werden.

Erfindungsgemäß ist weiterhin, dass die erfindungsgemäßen Zubereitungen Haut- und/oder Haarpflegesubstanzen enthalten, ausgewählt aus Glycerin, pflanzlichen Ölen, Paraffinölen oder Panthenol, einzeln oder in Kombination. Weiterhin können Silikone, kationische Polymere, Emollients, einzeln oder Kombination eingesetzt werden. Die Haut- und/oder Haarpflegesubstanzen sind mit einem Gehalt von 0,01 bis 10 Gew.-%, bevorzugt von 0,1 bis 8 Gew.-%, besonders bevorzugt von 0,2 bis 5 Gew.-% enthalten.

Erfindungsgemäß ist es, wenn die erfindungsgemäßen Zubereitungen Salze enthalten, wobei Alkalimetallsalze bevorzugt sind, insbesondere Alkalimetallhalogenide, Alkalimetallsulfate, Alkalimetallnitrate und/oder Alkalimetallphosphate. Das Salz wird oder die Salze werden in Konzentrationen von 0,1 bis 3,0 Gew.-% eingesetzt.

Erfindungsgemäß besonders bevorzugt sind erfindungsgemäße Zubereitungen, die anionische Tenside mit einem Gehalt von 1,0 bis 15 Gew.-%, bevorzugt 1,0 bis < 12 Gew.-% und hautpflegende Substanzen, bevorzugt Glycerin, pflanzliche Öle, Paraffinöle oder Panthenol, einzeln oder Kombination, enthalten.

Es ist erfindungsgemäß bevorzugt, wenn die erfindungsgemäßen Zubereitungen Substanzen enthalten, die speziell für die Anwendung auf Haaren und Kopfhaut geeignet sind, beispielsweise Substanzen zur Pflege und Konditionierung der Haare, wie auch Stoffe mit spezifischen Wirkungen, wie beispielsweise Antischuppenwirkstoffen.

Vorteilhaft sind auch Duschgele, Gesichtsreiniger, Shampoos oder kosmetische Hydrogele enthaltend ein oder mehrere AMA-X-Polymere.

Vorteilhaft ist auch die Verwendung der oben beschriebenen Zubereitungen zur kosmetischen Anwendung.

Vorteilhaft ist auch die Verwendung der oben beschriebenen Zubereitungen zur Reinigung der Haut, insbesondere der menschlichen Haut.

Vorteilhaft ist ebenfalls die Verwendung der oben beschriebenen Zubereitungen zur Anwendung auf Haaren, insbesondere zur Reinigung von Haaren, besonders bevorzugt zur Reinigung von menschlichen Haaren.

Vorteilhaft ist auch die Verwendung von AMA-X-Polymeren in wässrigen, tensidhaltigen Zubereitungen, wobei Werte für tan delta von 0,05 bis 0,6, bevorzugt 0,1 bis 0,5 bestimmt werden.

Vorteilhaft ist auch ein Verfahren zur Verminderung der Reizung der Haut während des Reinigungsvorganges wobei die Reinigungszubereitungen
- transparent sind, gekennzeichnet durch Trübungswerte unter 30 NTU, bevorzugt unter 25 NTU, besonders bevorzugt unter oder gleich 20 NTU liegen,
- ein oder mehrere vernetzte Acrylat Copolymere, insbesondere AMA-X-Polymere, mit einem Gehalt von 0,1 bis 5,0 %, bevorzugt 0,5 bis 4,0 %, besonders bevorzugt 1,0 bis 2,0 %, bezogen auf den Aktivgehalt und das Gesamtgewicht der Zubereitung,
- optional Partikel, Tröpfchen und/oder Blasen nahezu homogen in den Zubereitungen verteilt,
- optional weitere Struktur-bildende Polymere, mit einem Gehalt an AMA-X-Polymeren und Struktur-bildenden Polymeren von 0,1 bis 5,0 %, bevorzugt 0,2 bis 4,0%, besonders bevorzugt 0,5 bis 2,0 % beträgt, bezogen auf den Aktivgehalt,
- optional 0,1 bis 3,0 Gew.-% eines anorganischen Salzes, ausgewählt aus der Gruppe der Alkalimetallhalogenide, der Alkalimetallsulfate, der Alkalimetallnitrate und der Alkalimetallphosphate enthalten
- einen pH-Wert von 4,0 bis 7,0, bevorzugt 4,0 bis < 6,4, besonders bevorzugt 4,0 bis ≤ 5,5 und
- eine Viskosität ≥ 2000 mPa·s aufweisen,
- anionische Tenside mit einem Gehalt von 1,0 bis 15 Gew.-%, bevorzugt < 12 Gew.-% und
- optional Hautpflegesubstanzen mit einem Gehalt von 0,01 bis 10 Gew.-% enthalten.

Vorteilhaft ist auch ein Verfahren zur Reinigung von sensibler, empfindlicher und trockener Haut, wobei die Reinigungszubereitungen
- transparent sind, gekennzeichnet durch Trübungswerte unter 30 NTU, bevorzugt unter 25 NTU, besonders bevorzugt unter oder gleich 20 NTU liegen,
- ein oder mehrere vernetzte Acrylat Copolymere, insbesondere AMA-X-Polymere, mit einem Gehalt von 0,1 bis 5,0 %, bevorzugt 0,5 bis 4,0 %, besonders bevorzugt 1,0 bis 2,0 %, bezogen auf den Aktivgehalt und das Gesamtgewicht der Zubereitung,
- optional Partikel, Tröpfchen und/oder Blasen nahezu homogen in den Zubereitungen verteilt,
- optional weitere Struktur-bildende Polymere, mit einem Gehalt an AMA-X-Polymeren und Struktur-bildenden Polymeren von 0,1 bis 5,0 %, bevorzugt 0,2 bis 4,0%, besonders bevorzugt 0,5 bis 2,0 % beträgt, bezogen auf den Aktivgehalt,
- optional 0,1 bis 3,0 Gew.-% eines anorganischen Salzes, ausgewählt aus der Gruppe der Alkalimetallhalogenide, der Alkalimetallsulfate, der Alkalimetallnitrate und der Alkalimetallphosphate enthalten
- einen pH-Wert von 4,0 bis 7,0, bevorzugt 4,0 bis < 6,4, besonders bevorzugt 4,0 bis ≤ 5,5 und
- eine Viskosität ≥ 2000 mPa·s aufweisen,
- anionische Tenside mit einem Gehalt von 1,0 bis 15 Gew.-%, bevorzugt < 12 Gew.-% und
- optional Hautpflegesubstanzen mit einem Gehalt von 0,01 bis 10 Gew.-% enthalten.

Vorteilhaft ist auch ein Verfahren zur Verminderung des Austrocknens der Haare und/oder des Entstehens von spröden Haaren wobei Reinigungszubereitungen
- transparent sind, gekennzeichnet durch Trübungswerte unter 30 NTU, bevorzugt unter 25 NTU, besonders bevorzugt unter oder gleich 20 NTU liegen,
- ein oder mehrere vernetzte Acrylat Copolymere, insbesondere AMA-X-Polymere, mit einem Gehalt von 0,1 bis 5,0 %, bevorzugt 0,5 bis 4,0 %, besonders bevorzugt 1,0 bis 2,0 %, bezogen auf den Aktivgehalt und das Gesamtgewicht der Zubereitung,
- optional Partikel, Tröpfchen und/oder Blasen nahezu homogen in den Zubereitungen verteilt,
- optional weitere Struktur-bildende Polymere, mit einem Gehalt an AMA-X-Polymeren und Struktur-bildenden Polymeren von 0,1 bis 5,0 %, bevorzugt 0,2 bis 4,0%, besonders bevorzugt 0,5 bis 2,0 % beträgt, bezogen auf den Aktivgehalt,
- optional 0,1 bis 3,0 Gew.-% eines anorganischen Salzes, ausgewählt aus der Gruppe der Alkalimetallhalogenide, der Alkalimetallsulfate, der Alkalimetallnitrate und der Alkalimetallphosphate enthalten
- einen pH-Wert von 4,0 bis 7,0, bevorzugt 4,0 bis < 6,4, besonders bevorzugt 4,0 bis ≤ 5,5 und
- eine Viskosität ≥ 2000 mPa·s aufweisen,
- anionische Tenside mit einem Gehalt von 1,0 bis 15 Gew.-%, bevorzugt < 12 Gew.-% und
- optional Haut- und/oder Haarpflegesubstanzen mit einem Gehalt von 0,01 bis 10 Gew.-% enthalten.

Auch vorteilhaft ist ein Verfahren zur Pflege der Haut und/oder Haare während des Reinigungsvorganges wobei Reinigungszubereitungen
- transparent sind, gekennzeichnet durch Trübungswerte unter 30 NTU, bevorzugt unter 25 NTU, besonders bevorzugt unter oder gleich 20 NTU liegen,
- ein oder mehrere vernetzte Acrylat Copolymere, insbesondere AMA-X-Polymere, mit einem Gehalt von 0,1 bis 5,0 %, bevorzugt 0,5 bis 4,0 %, besonders bevorzugt 1,0 bis 2,0 %, bezogen auf den Aktivgehalt und das Gesamtgewicht der Zubereitung,
- optional Partikel, Tröpfchen und/oder Blasen nahezu homogen in den Zubereitungen verteilt,
- optional weitere Struktur-bildende Polymere, mit einem Gehalt an AMA-X-Polymeren und Struktur-bildenden Polymeren von 0,1 bis 5,0 %, bevorzugt 0,2 bis 4,0%, besonders bevorzugt 0,5 bis 2,0 % beträgt, bezogen auf den Aktivgehalt,
- optional 0,1 bis 3,0 Gew.-% eines anorganischen Salzes, ausgewählt aus der Gruppe der Alkalimetallhalogenide, der Alkalimetallsulfate, der Alkalimetallnitrate und der Alkalimetallphosphate enthalten
- einen pH-Wert von 4,0 bis 7,0, bevorzugt 4,0 bis < 6,4, besonders bevorzugt 4,0 bis ≤ 5,5 und
- eine Viskosität ≥ 2000 mPa·s aufweisen,
- anionische Tenside mit einem Gehalt von 1,0 bis 15 Gew.-%, bevorzugt < 12 Gew.-% und
- Haut- und/oder Haarpflegesubstanzen mit einem Gehalt von 0,01 bis 10 Gew.-% enthalten.

Ebenso vorteilhaft sind Verfahren zur Verminderung der Reizung der Haut während des Reinigungsvorganges, zur Reinigung von sensibler, empfindlicher und trockener Haut und/oder zur Verminderung des Austrocknens der Haare und/oder des Entstehens von spröden Haaren, wobei die Reinigungszubereitungen
- transparent sind, gekennzeichnet durch Trübungswerte unter 30 NTU, bevorzugt unter 25 NTU, besonders bevorzugt unter oder gleich 20 NTU liegen,
- ein oder mehrere vernetzte Acrylat Copolymere, insbesondere AMA-X-Polymere, mit einem Gehalt von 0,1 bis 5,0 %, bevorzugt 0,5 bis 4,0 %, besonders bevorzugt 1,0 bis 2,0 %, bezogen auf den Aktivgehalt und das Gesamtgewicht der Zubereitung,
- optional Partikel, Tröpfchen und/oder Blasen nahezu homogen in den Zubereitungen verteilt,
- optional weitere Struktur-bildende Polymere, mit einem Gehalt an AMA-X-Polymeren und Struktur-bildenden Polymeren von 0,1 bis 5,0 %, bevorzugt 0,2 bis 4,0%, besonders bevorzugt 0,5 bis 2,0 % beträgt, bezogen auf den Aktivgehalt,
- optional 0,1 bis 3,0 Gew.-% eines anorganischen Salzes, ausgewählt aus der Gruppe der Alkalimetallhalogenide, der Alkalimetallsulfate, der Alkalimetallnitrate und der Alkalimetallphosphate enthalten
- einen pH-Wert von 4,0 bis 7,0, bevorzugt 4,0 bis < 6,4, besonders bevorzugt 4,0 bis ≤ 5,5 und
- eine Viskosität ≥ 2000 mPa·s aufweisen,
- anionische Tenside mit einem Gehalt von 1,0 bis 15 Gew.-%, bevorzugt < 12 Gew.-% und
- optional Haut- und/oder Haarpflegesubstanzen mit einem Gehalt von 0,01 bis 10 Gew.-% enthalten und
- eine Milde, gemessen als L/D-Quotient im RBC-Assay, mit Werten ≤ 0,4, bevorzugt ≤ 0,35 aufweisen.

Die Viskositätswerte, die in der vorliegenden Schrift offenbart werden, sind mit dem Rheomat R123 der Gesellschaft ProRheo bei 25°C gemessen worden. Bei der Messung mit dem Rheomat R123 wird der Rotor des Gerätes blasenfrei bis zur Markierung in die Probe eingetaucht. Für die Messungen wurde der Messkörper 1 verwendet. Weitergehende Informationen zum Rheomat R 123 sind im Internet veröffentlicht, siehe
http://www.prorheo.de/fileadmin/user_upload/pdfs/R123.pdf und http://www.prorheo.de/fileadmin/user_upload/pdfs/Bedienung_R123_d.pdf.

Die Formulierungen weisen einen tan-delta von ≤0,5 auf, wodurch eine Stabilisierung von Partikeln gegeben ist. Unter dem tan-delta wird der Quotient aus dem Verlustmodul und dem Speichermodul verstanden. Der tan-delta wird wie folgt ermittelt:
Gemessen werden Verlust-und Speichermodul durch einen dynamischen, schubspannungsgesteuertem Frequenztest bei einer Schubspannung von 1 Pa auf einem schubspannungsgesteuerten Rheometer (SR-Serie der Fa. Rheometric Scientific oder AR-Serie der Fa. TA-Instruments oder andere) bei 40°C ± 1°C mit 25 mm Platte/Platte Geometrie bei einem Spalt zwischen 0,8 mm und 1,2 mm, wobei strukturschonend befüllt wird. Es wird nach dem Stand der Technik der Frequenztest mit einer entsprechenden Strukturerholungszeit vor dem Test durchgeführt und der tan-delta im Frequenzbereich zwischen 0,05 rad/s und 3,0 rad/s angegeben, bevorzugt zwischen 0,08 rad/s und 1,0 rad/s.

Als Fließgrenze wird die kritische Schubspannung der Fließkurve angesehen. Sie kann erfindungsgemäß wie folgt ermittelt werden:
Gemessen wird die Fließkurve auf einem schubspannungsgesteuerten Rheometer bei 25°C ± 1 °C mit 20 mm Platte/Platte Geometrie bei einem Spalt zwischen 0,8 mm und 1,2 mm, wobei strukturschonend befüllt wird. Es wird eine geeignete konstante Schubspannungszeitrampe vorgegeben und vor dem Test eine entsprechende Strukturerholungszeit eingehalten und die kritische Schubspannung im Maximum der Fließkurve angegeben.

Für die Bestimmung der Milde wird ein RBC-Assay durchgeführt.

Der Standard-RBC-Assay (10 Minuten Inkubation) dient zur Abschätzung von in vivo Augenschleimhautreizpotentialen von Tensiden und tensidhaltigen Produkten.

### 1. Hämolyse

Ein definiertes Aliquot isolierter Kalbserythrozyten wird mit einer Reihe steigender Konzentrationen des zu untersuchenden WAS-Prüfmusters (Stammlösung mit Formulierungen 1:100 w/v bzw. für Rohstoffe 0.1 % Aktivgehalt in PBS) für 10 Minuten unter Schütteln bei Raumtemperatur (RT) inkubiert. Nach Zentrifugation werden die gewonnenen Überstände photometrisch auf ihren Gehalt an freigesetztem Hämoglobin (HbO₂) bei 530 nm analysiert. Daraus wird der relative Hämolysegrad berechnet und die Konzentrations-Response-Kurve mit dem H50-Wert [µl/ml] als Kenngröße bestimmt. Dieser gibt die Konzentration des Prüfmusters an, bei der 50% des Hämoglobins freigesetzt werden.

### 2. HbO₂-Denaturierung

Ein definiertes Aliquot isolierter Kalbserythrozyten wird mit einer fixen Konzentration des Prüfmusters (1% w/v bzw. 0.1% Aktivgehalt) für 10 Minuten unter Schütteln bei RT inkubiert und dann zentrifugiert. Die Änderung der spektralen Absorption bei 575 nm und 540 nm wird im Vergleich zum nativen HbO₂ gemessen. Aus dem Verhältnis der Absorptionswerte zueinander wird der Denaturierungsindex DI [%] berechnet. Als 100%-Standard dient Na-Laurylsulfat (0.1% Aktivgehalt).

### 3. L/D-Quotient

Der Quotient stellt das Verhältnis der Kenngrößen von Hämolyse (H50) und Denaturierung (DI) dar und wird zur Charakterisierung und Klassifizierung der untersuchten Prüfmuster verwendet.

Trübungswerte werden gemessen mit einem Trübungsmessgerät, wobei destilliertes Wasser mit einem Wert von NTU=0 als Standard gilt.

### Weitere Struktur-bildende Polymere:

Zur Verdickung und Stabilisierung von Partikeln können optional weitere Polymere eingesetzt werden, die ausgewählt werden können aus der Gruppe Polysaccharide bzw. deren Derivate, z. B. Hyaluronsäure, Hydroxypropylmethylcellulose, besonders vorteilhaft aus der Gruppe der Polyacrylate, bevorzugt ein Polyacrylat aus der Gruppe der so genannten Carbopole, beispielsweise Carbopole der Typen 980, 981, 1382, 2984, 5984, Ultrez 2020, Ultrez 10 oder Pemulen TR1 oder auch ethoxylierte Glycerin-Fettsäureester und deren Derivate wie beispielsweise Hydrogenated Glyceryl Palmate, PEG-90 Glyceryl Isostearat, jeweils einzeln oder in Kombination.

### Tenside:

Die Tenside, die in den erfindungsgemäßen Zubereitungen Verwendung finden, können anionische Tenside in Kombination mit amphoteren, nichtionischen und/oder kationischen Tensiden sein.

### Vorteilhaft zu verwendende anionische Tenside sind

### Acylaminosäuren (und deren Salze), wie

1. Acylglutamate, beispielsweise Natriumacylglutamat, Natrium Cocoylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-Iauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

### Carbonsäuren und Derivate, wie

1. Carbonsäuren, beispielsweise Laurinsäure, Aluminiumstearat, Magnesiumalkanolat und Zinkundecylenat, Stearinsäure/-salz, Palmitinsäure/-salz,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,
Phosphorsäureester und Salze, wie beispielsweise DEA-Oleth-10-Phosphat und Dilaureth-Phosphat,

### Sulfonsäuren und Salze, wie

1. Acyl-isethionate, z.B. Natrium-/ Ammoniumcocoyl-isethionat, Natrium Lauryl Methylisethionate,
2. Alkylarylsulfonate,
3. Alkylsulfonate, beispielsweise Natriumcocosmonoglyceridsulfat, Natrium C12-14 Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie

### Schwefelsäureester, wie

1. Alkylethersulfat mit unterschiedlichen Ethoxylierungsgraden und deren Gemische, beispielsweise Natrium-, Ammonium-, Magnesium-, MIPA-, TIPA-Laureth-X-sulfat, Natriummyreth-X-sulfat und Natrium C12-13-Pareth-X-sulfat, mit X = 1-5 Ethoxygruppen.
2. Alkylsulfate, beispielsweise Natrium-, Ammonium- und TEA-Laurylsulfat, Natrium-Ammonium- und TEA-Cocosulfat.

### Vorteilhaft zu verwendende kationische Tenside sind

1. Alkylamine,
2. Alkylimidazole,
3. Ethoxylierte Amine
4. Quaternäre Tenside, beispielsweise Cetyl Trimethylammoniumhalogenid.
5. Esterquats, beispielsweise Dicocoylethyl Hydroxyethylmoium Methosulfate und
6. Amidquats, beispielsweise Palmitamidopropyltrimonium Chlorid.

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkylamidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
3. Betaine, beispielsweise Coco Betaine, Cocoamidopropyl Betaine,
4. Sultaine, beispielsweise Lauryl Hydroxy Sultaine.

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, Laureth-X mit X = 2 bis 10, wobei X Ethoxygruppe bedeutet, ethoxylierte/ propoxylierte Ester, ethoxylierte/ propoxylierte Glycerinester, PEG-200 Hydrogenated Glyceryl Palmat, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

### UV-Filter:

Es ist vorteilhaft im Sinne der vorliegenden Erfindungen, den Zubereitungen Sonnenschutzfilter zu zusetzen. Der hauptsächliche Zweck dieser Zubereitungen ist jedoch nicht der Schutz vor Sonnenlicht ist, sie enthalten aber gleichwohl einen Gehalt an UV-Schutzsubstanzen.

Es ist vorteilhaft im Sinne der vorliegenden Erfindung, wenn die verwendeten UVFiltersubstanzen wasserlöslich sind.

Erfindungsgemäße wasserlösliche UV-Filtersubstanzen sind z. B.:
- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Ferner können an Polymere gebundene öllösliche UV-Filter, wie z. B. Polysilicone-15, das auch unter dem Handelsnamen Parsol SLX erhältlich ist.

Es ist bevorzugt im Sinne der vorliegenden Erfindung beispielsweise Benzophenone-4 zu verwenden.

Die Gesamtmenge der Filtersubstanzen wird aus dem Bereich von 0,01 bis 30 Gew.-%, vorzugsweise 0,02 bis 10 Gew.-%,-jeweils bezogen auf das Gesamtgewicht der Zubereitungen - gewählt.

### Lösungsmittel:

Die erfindungsgemäßen Zubereitungen können gegebenenfalls vorteilhaft Alkohole, Diole oder Polyole niedriger C-Zahl, sowie deren Ether, vorzugsweise Ethanol, Isopropanol, 1,2-Propandiol, Propylenglykol, Ethylenglykol, Ethylenglykolmonoethyl- oder-monobutylether, Propylenglykolmonomethyl, -monoethyl- oder -monobutylether, Diethylenglykolmonomethyl- oder -monoethylether und analoge Produkte enthalten.

### Konservierungsmittel:

Zur Konservierung der vorliegenden Zubereitungen können die in der Kosmetik üblicherweise verwendeten Konservierungsmittel eingesetzt werden. Dazu zählen beispielsweise Parabene, wie Methylparaben, Propylparaben, Ethylparaben und Butylparaben. Ebenso können andere Konservierungsstoffe zum Einsatz kommen, wie beispielsweise Methylisothiazolinone und Hydantoine wie beispielsweise DMDM Hydantoine. Wünschenswert ist aber auch der Einsatz von Konservierungsmitteln auf Säure-Basis, die in der Nahrungsmittelindustrie zum Einsatz kommen. Hierzu zählen beispielsweise Benzoesäure und/oder Salicylsäure und/oder ihre Salze. Da diese Konservierungsmittel ihre Wirkung in einem sauren pH-Wert-Bereich entfalten, einem pH-Bereich der für die menschliche Haut (pH-Wert der menschlichen Haut 5,4 bis 5,9, in einigen Hautarealen sogar unter pH-Wert = 5,0) vorteilhaft und für viele Bakterien unvorteilhaft ist, ist der Einsatz dieser Konservierungsmittel von großem Vorteil.

### Hautpflegesubstanzen:

Es ist vorteilhaft, den erfindungsgemäßen Zubereitungen Substanzen zu zusetzen, welche die Pflege der Haut unterstützen, die Haut vor Austrocknung schützen, Irritationen reduzieren, die Haut geschmeidiger und weicher machen und das Aussehen der Haut verbessern. Dazu zählen Glycerin, Panthenol, Fettsäuren mit einer Kettenlänge von C8 bis C22, Fettalkohole mit einer Kettenlänge von C14 bis C22, Paraffinöle sowie pflanzliche Öle.

### Haarpfleaesubstanzen:

Neben den beschriebenen Hautpflegesubstanzen, die auch dem Haar ein gewisses Maß an Pflege geben, was sich u.a. durch bessere Frisierbarkeit und einen angenehmen Griff bemerkbar macht, können auch Silikone, kationische Polymere und weitere Haarpflegesubstanzen den erfindungsgemäßen Zubereitungen zugesetzt werden.

### pH-Wert Einstellung:

Die Einstellung des pH-Wertes kann auf die in der kosmetischen Industrie üblichen Art und Weise erfolgen. Bevorzugt ist jedoch der Einsatz von Zitronensäure und Natriumhydroxid um den erforderlichen pH-Wert einzustellen.

### Salze:

Die erfindungsgemäßen Zubereitungen können vorteilhaft auch Salze enthalten. Diese Salze sind bevorzugt anorganische Salze. Besonders vorteilhaft sind Alkalimetallsalze wie Alkalihalogenide, Alkalisulfate, Alkalinitrate und Alkaliphosphate.

### Komplexbildner:

Es ist erfindungsgemäß ferner gegebenenfalls vorteilhaft, den Zubereitungen Komplexbildner zuzufügen. Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, dass die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen) und Tetrasodium Iminodisuccinate.

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

### Partikel:

Partikel im Sinne der vorliegenden Schrift sind Partikel aus allen organischen und anorganischen Feststoffen auf natürlicher und synthetischer Basis. Verwendet werden z.B. Kunststoffpartikel aus beispielsweise Viskose, Zellulose, Polypropylen, Polyester, Polyethylenterephthalat (PET), Polytetraflourethylen (PTFE), Aramid, Nylon, Kevlar, Polyurethane, Polystyrol, Celluloseester und/oder Polyethylen sowie alle Arten von Gesteinsmehl, gemahlene Pflanzenbestandteile wie beispielsweise Nussschalen und Kerne. Es kommen auch Mischungen verschiedener Partikel in Frage, die durch geeignete physikalische verfahren, wie z.B. Pressen pelletiert werden. Bevorzugt sind beispielsweise Unispheres® der Firma Induchem oder Cosmospheres® der Firma Pelletech.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen Zubereitungen können dementsprechend ferner kosmetische Hilfsstoffe enthalten, wie sie üblicherweise in solchen Zubereitungen verwendet werden, beispielsweise Parfüme, Substanzen zum Verhindern des Schäumens, Farbstoffe, Pigmente, die färbende Wirkung haben, Emulgatoren, weichmachende, entzündungshemmende Substanzen, Insektenrepellentien, Antischuppenwirkstoffe, Bakterizide, Viruzide, Salze, antimikrobiell, proteolytisch oder keratolytisch wirksame Substanzen oder andere übliche Bestandteile einer kosmetischen Formulierung wie Schaumstabilisatoren und Elektrolyte.

Durch den Einsatz von AMA-X-Polymeren konnten transparente Formulierungen in einem pH-Wert-Bereich von 4,0 bis 7,0, bevorzugt 4,0 bis < 6,4, besonders bevorzugt 4,0 bis ≤ 5,5, entwickelt werden. Transparente Formulierung bedeutet, dass die Trübungswerte unter 30 NTU, bevorzugt unter 25 NTU, besonders bevorzugt unter oder gleich 20 NTU liegen. Diese Formulierungen weisen geeignete Fließeigenschaften auf, gekennzeichnet durch tan delta Werte, die bevorzugt ≤ 0,5 sind, so dass feste Partikel, Tröpfchen und/oder Bläschen stabil suspendiert werden und es auch bleiben.

Da in den erfindungsgemäßen Zubereitungen der Gehalt an anionischen Tensiden unter 10 % liegt und eine Kombination von anionischen Tensiden mit amphoteren, nichtionischen und/oder kationischen Tensiden eingesetzt werden kann, zeichnen sich diese Zubereitungen dadurch aus, dass sie milde sind. Diese derart hergestellten Formulierungen haben Trübungswerte < 30 NTU, bevorzugt <25 NTU, besonders bevorzugt ≤ 20 NTU und einen bevorzugten tans delta ≤ 0,5. Der Einsatz von Hautpflegesubstanzen erhöht die Milde dieser Zubereitungen darüber hinaus noch einmal. Dies wird deutlich durch einen Vergleichsversuch (siehe Beispiele, Vergleichsversuch zur Bestimmung der Milde), bei dem ein Handelsprodukt mit einer erfindungsgemäßen Zubereitung hinsichtlich der Milde verglichen wurde. Das eingesetzte Handelsprodukt ist Balea Dusche &Ölperlen Queen of the Night, diese Zubereitung enthält als Struktur-gebendes Polymer Aqua SF2 von der Firma Lubrizol. Die erfindungsgemäße Zubereitung erreicht in Bezug auf die Milde, gemessen mit dem Standard RBC-Test (WAS), bessere Werte als das Handelsprodukt. Die Ergebnisse sind im Vergleichsversuch zur Bestimmung der Milde unter Beispielen aufgeführt. Da die erfindungsgemäßen Zubereitungen sich als milde Zubereitungen ausweisen, sind die besonders geeignet, um eine Verminderung der Reizung der Haut während des Reinigungsvorganges zu erzielen, zur Reinigung von sensibler, empfindlicher und trockener Haut und um eine Verminderung des Austrocknens der Haare zu erreichen und/oder dem Entstehens von spröden Haaren entgegen zu wirken.

Darüber hinaus sind die erfindungsgemäßen Zubereitungen überaus befriedigende Produkte im Hinblick auf das Aussehen der Produkte. Es werden keine "Polymer-Wölkchen" sichtbar, wie sie bei Produkten, die als Struktur-gebendes Polymer Aqua SF2 (Firma Lubrizol) enthalten, unter bestimmten Bedingungen zu beobachten sind. Ebenso sind in den erfindungsgemäßen Zubereitungen keine Polymerausfällungen zu sehen. Diese Polymerausfällungen, die bei Produkten beobachtet werden können, die AquaSF2 enthalten, führen zu Anhaftungen von Produkt und/oder Polymer an den Wandungen der Packmittel. Die erfindungsgemäßen Zubereitungen sind gleichmäßig transparente Zubereitungen mit einem äußerst befriedigenden Fließverhalten.

Die nachfolgenden Beispiele verdeutlichen die Erfindung ohne sie jedoch einzuschränken.

### Beispiele:

### Duschgele (die Mengenangaben sind Aktivgehalte):

| **Beispiel Nr.** | **1***** | **2** | **3***** | **4***** | **5** | **6***** | **7***** |
|---|---|---|---|---|---|---|---|
| Sodium Laureth Sulfate | 6,5 | 8,5 | 9,5 | 8,5 | 9,0 | 8,75 | 7,0 |
| Cocamidopropyl Betaine | 3,0 | 3,0 | | 3,2 | 3,5 | 2,9 | 2,8 |
| Sodium Myreth Sulfate | 3,0 | | | | | | 2,5 |
| Disodium Cocoyl Glutamate | | | | 0,5 | | | |
| Decyl Glucoside | | 1,0 | | | | | 1,0 |
| Coco Glucoside | | | 1,0 | | | | |
| Coco Betaine | | | 2,0 | | | | |
| AMA-X-Polymer | 1,44 | 1,30 | 1,28 | 1,28 | 1,30 | 1,44 | 1,40 |
| PEG-7 Glyceryl Cocoate | 1,0 | 1,0 | 1,5 | 1,0 | 1,75 | 2,0 | 1,0 |
| PEG-40 Hydrogenated Castor Oil | 0,8 | 0,6 | 0,6 | 0,6 | 0,8 | 0,6 | 0,7 |
| Benzophenone-4 | | 0,05 | | 0,02 | 0,05 | | |
| Glycerin | | | | | 1,0 | | |
| Sodium Benzoate | 0,5 | 0,45 | 0,4 | 0,45 | 0,45 | 0,4 | 0,5 |
| Sodium Salicylate | | | 0,2 | | 0,1 | 0,15 | |
| **Helianthus Annuus Seed Oil** | | 0,1 | | | 0,1 | | 0,01 |
| Cosmospheres®* | 0,05 | 0,08 | | 0,15 | 0,08 | | |
| Unispheres®** | | | 0,1 | | | 0,2 | 0,15 |
| Polyethylen particle | 0,1 | | | 0,2 | | | |
| Cl 42090 | | | 0,0001 | | | 0,002 | |
| Cl 10316 | | | 0,002 | | | | |
| Citric Acid | q.s. | q.s. | q.s. | q.s. | q.s. | q.s. | q.s. |
| Sodium Hydroxide | q.s. | q.s. | q.s. | q.s. | q.s. | q.s. | q.s. |
| Parfuem | 1,0 | 0,9 | 1,0 | 0,8 | 0,85 | 1,0 | 1,0 |
| Aqua | ad 100 | ad 100 | ad 100 | ad 100 | ad 100 | ad 100 | ad 100 |

| | | | | | | | |
|---|---|---|---|---|---|---|---|
| *** Nicht erfindungsgemäßes Beispiel. | | | | | | | |

### Duschgele (Die Mengenangaben sind Aktivgehalte):

| **Beispiel Nr.** | **8** | **9***** | **10** | **11** | **12***** | **13***** | **14** |
|---|---|---|---|---|---|---|---|
| Sodium Laureth Sulfate | 8,0 | 8,5 | 8,5 | 7,5 | 9,0 | 8,5 | 9,5 |
| Cocamidopropyl Betaine | 3,0 | 3,0 | | 3,0 | 3,0 | 2,9 | |
| Sodium Myreth Sulfate | | | | 2,0 | | | |
| Disodium Cocoyl Glutamate | 1,0 | | 0,5 | | | | |
| Decyl Glucoside | | | 1,0 | | | 0,5 | |
| Coco Glucoside | | 1,5 | | | 0,8 | 0,8 | 1,0 |
| Coco Betaine | | | 2,0 | | | | 2,0 |
| AMA-X-Polymer | 1,44 | 1,28 | 1,28 | 1,32 | 1,40 | 1,60 | 1,28 |
| PEG-7 Glyceryl Cocoate | 1,2 | 1,0 | 1,0 | 1,0 | | 1,1 | 1,0 |
| PEG-40 Hydrogenated Castor Oil | 0,65 | 0,6 | 0,7 | 0,6 | 0,8 | 0,65 | 0,6 |
| Benzophenone-4 | | 0,05 | | 0,05 | 0,06 | 0,05 | 0,03 |
| Glycerin | | | 0,5 | | | | |
| Sodium Benzoate | 0,4 | 0,45 | 0,5 | 0,45 | 0,35 | 0,40 | 0,45 |
| Sodium Salicylate | 0,1 | | | | 0,40 | 0,10 | |
| **Helianthus Annuus Seed Oil** | 0,01 | | | 0,1 | | | 0,1 |
| PEG-200 Hydrogenated Glyceryl Palmate | | 0,8 | | | 0,2 | | |
| Cosmospheres®* | 0,2 | | | 0,15 | | | |
| Unispheres®** | | 0,13 | | | | 0,11 | 0,08 |
| Polyethylen particle | | | 0,3 | | 0,09 | 0,05 | |
| Cl 42090 | | | | | | 0,0001 | |
| Cl 15985 | | 0,0005 | | | | | |
| Citric Acid | q.s. | q.s. | q.s. | q.s. | q.s. | q.s. | q.s. |
| Sodium Hydroxide | q.s. | q.s. | q.s. | q.s. | q.s. | q.s. | q.s. |
| Parfuem | 1,0 | 0,8 | 0,9 | 1,0 | 1,1 | 0,85 | 1,0 |
| Aqua | ad 100 | ad 100 | ad 100 | ad 100 | ad 100 | ad 100 | ad 100 |

| | | | | | | | |
|---|---|---|---|---|---|---|---|
| *** Nicht erfindungsgemäßes Beispiel. | | | | | | | |

### Duschgele (Die Mengenangaben sind Aktivgehalte):

| **Beispiel Nr.** | **15** | **16***** | **17***** | **18** | **19** | **20***** |
|---|---|---|---|---|---|---|
| Sodium Laureth Sulfate | 9,0 | 9,0 | 8,5 | 7,0 | 6,5 | 7,5 |
| Cocamidopropyl Betaine | 3,0 | 3,0 | 3,1 | 3,0 | 3,2 | 3,0 |
| Sodium Myreth Sulfate | | | | 2,0 | 3,0 | |
| Disodium Cocoyl Glutamate | | | 0,5 | | | 1,0 |
| Decyl Glucoside | | 1,0 | 0,2 | | | 0,3 |
| Coco Glucoside | | | 0,3 | 0,5 | 0,6 | 0,4 |
| AMA-X-Polymer | 1,28 | 1,44 | 1,45 | 1,40 | 1,35 | 1,44 |
| PEG-7 Glyceryl Cocoate | 1,0 | 1,5 | 1,2 | 1,0 | 1,1 | |
| PEG-40 Hydrogenated Castor Oil | 0,6 | 0,6 | 0,8 | 0,7 | 0,65 | 0,6 |
| Benzophenone-4 | 0,05 | 0,05 | | 0,04 | | |
| Glycerin | | | | | 1,0 | |
| Sodium Benzoate | 0,45 | 0,45 | 0,40 | 0,35 | 0,50 | 0,4 |
| Sodium Salicylate | | | 0,10 | 0,15 | | 0,1 |
| **Helianthus Annuus Seed Oil** | 0,1 | | | 0,01 | | |
| Cosmospheres®* | | 0,08 | | | 0,07 | |
| Unispheres®** | 0,1 | | 0,15 | | | 0,13 |
| Polyethylen particle | | | | 0,1 | | |
| Cl 10316 | | | 0,002 | | | |
| Cl 16035 | | | | | 0,0004 | |
| Citric Acid | q.s. | q.s. | q.s. | q.s. | q.s. | q.s. |
| Sodium Hydroxide | q.s. | q.s. | q.s. | q.s. | q.s. | q.s. |
| Parfuem | 1,0 | 0,9 | 0,85 | 1,0 | 1,0 | 1,3 |
| Aqua | ad 100 | ad 100 | ad 100 | ad 100 | ad 100 | ad 100 |

| | | | | | | |
|---|---|---|---|---|---|---|
| *** Nicht erfindungsgemäßes Beispiel. | | | | | | |

### Duschgele (Die Mengenangaben sind Aktivgehalte):

| **Beispiel Nr.** | **21** | **22** | **23***** | **24** | **25** | **26** |
|---|---|---|---|---|---|---|
| Sodium Laureth Sulfate | 9,0 | 9,0 | 8,5 | 7,0 | 6,5 | 7,5 |
| Cocamidopropyl Betaine | 3,0 | 3,0 | 3,1 | 3,0 | 3,2 | 3,0 |
| Sodium Myreth Sulfate | | | | 2,0 | 3,0 | |
| Disodium Cocoyl Glutamate | | | 0,5 | | | 1,0 |
| Decyl Glucoside | | 1,0 | 0,2 | | | 0,3 |
| Coco Glucoside | | | 0,3 | 0,5 | 0,6 | 0,4 |
| AMA-X-Polymer | 1,28 | 1,44 | 1,45 | 1,40 | 1,35 | 1,44 |
| PEG-7 Glyceryl Cocoate | 1,0 | 1,5 | 1,2 | 1,0 | 1,1 | |
| PEG-40 Hydrogenated Castor Oil | 0,6 | 0,6 | 0,8 | 0,7 | 0,65 | 0,6 |
| Benzophenone-4 | 0,05 | 0,05 | | 0,04 | | |
| Glycerin | | | | | 1,0 | |
| Sodium Benzoate | 0,45 | 0,45 | 0,40 | 0,35 | 0,50 | 0,4 |
| Sodium Salicylate | | | 0,10 | 0,15 | | 0,1 |
| **Glycine Soja** | 0,1 | | | 0,01 | 0,1 | 0,05 |
| **Mineral Oil** | | 0,05 | | | | 0,05 |
| **Ricinus Communis** | | | 0,08 | | 0,01 | |
| Cosmospheres®* | | 0,08 | | | 0,07 | |
| Unispheres®** | 0,1 | | 0,15 | | | 0,13 |
| Polyethylen particle | | | | 0,1 | | |
| Cl 10316 | | | 0,002 | | | |
| Cl 16035 | | | | | 0,0004 | |
| Citric Acid | q.s. | q.s. | q.s. | q.s. | q.s. | q.s. |
| Sodium Hydroxide | q.s. | q.s. | q.s. | q.s. | q.s. | q.s. |
| Parfuem | 1,0 | 0,9 | 0,85 | 1,0 | 1,0 | 1,3 |
| Aqua | ad 100 | ad 100 | ad 100 | ad 100 | ad 100 | ad 100 |

| | | | | | | |
|---|---|---|---|---|---|---|
| *** Nicht erfindungsgemäßes Beispiel. | | | | | | |

### Duschgele (Die Mengenangaben sind Aktivgehalte):

| **Beispiel Nr.** | **27** | **28** | **29** | **30** | **31** | **32** |
|---|---|---|---|---|---|---|
| Sodium Laureth Sulfate | 9,0 | 9,0 | 8,5 | 7,0 | 6,5 | 7,5 |
| Cocamidopropyl Betaine | 3,0 | 3,0 | 3,1 | 3,0 | 3,2 | 3,0 |
| Sodium Myreth Sulfate | | | | 2,0 | 3,0 | |
| Disodium Cocoyl Glutamate | | | 0,5 | | | 1,0 |
| Decyl Glucoside | | 1,0 | 0,2 | | | 0,3 |
| Coco Glucoside | | | 0,3 | 0,5 | 0,6 | 0,4 |
| AMA-X-Polymer | 1,28 | 1,44 | 1,45 | 1,40 | 1,35 | 1,44 |
| PEG-7 Glyceryl Cocoate | 1,0 | 1,5 | 1,2 | 1,0 | 1,1 | |
| PEG-40 Hydrogenated Castor Oil | 0,6 | 0,6 | 0,8 | 0,7 | 0,65 | 0,6 |
| Benzophenone-4 | 0,05 | 0,05 | | 0,04 | | |
| Glycerin | | | | | 1,0 | |
| Sodium Benzoate | 0,45 | 0,45 | 0,40 | 0,35 | 0,50 | 0,4 |
| Sodium Salicylate | | | 0,10 | 0,15 | | 0,1 |
| **Glycine Soja** | 0,1 | | | 0,01 | 0,1 | 0,05 |
| **Mineral Oil** | | 0,05 | | | | 0,05 |
| **Ricinus Communis** | | | 0,08 | | 0,01 | |
| Cl 10316 | | | 0,002 | | | |
| Cl 16035 | | | | | 0,0004 | |
| Citric Acid | q.s. | q.s. | q.s. | q.s. | q.s. | q.s. |
| Sodium Hydroxide | q.s. | q.s. | q.s. | q.s. | q.s. | q.s. |
| Parfuem | 1,0 | 0,9 | 0,85 | 1,0 | 1,0 | 1,3 |
| Aqua | ad 100 | ad 100 | ad 100 | ad 100 | ad 100 | ad 100 |

### Shampoos (Die Mengenangaben sind Aktivgehalte):

| **Beispiel Nr.** | **1***** | **2***** | **3***** | **4***** | **5***** | **6***** |
|---|---|---|---|---|---|---|
| Sodium Laureth Sulfate | 8,5 | 11,0 | 9,5 | 7,5 | 10,0 | 11,7 |
| Cocamidopropyl Betaine | 3,0 | 3,2 | 3,8 | 2,2 | 3,1 | 4 |
| Cocamide DEA | | | | 2,0 | 1,0 | |
| Decyl Glucoside | | 1,0 | 0,2 | | | 0,3 |
| AMA-X-Polymer | 1,6 | 1,2 | 1,3 | 1,8 | 1,35 | 1,12 |
| PEG-40 Hydrogenated Castor Oil | 0,6 | 0,6 | 0,8 | 0,7 | 0,65 | 0,6 |
| Benzophenone-4 | 0,05 | 0,05 | 0,05 | 0,04 | 0,05 | 0,05 |
| Polyquaternium-10 | 0,3 | | | | 0,3 | |
| Guar Hydroxyproypltrimonium Chloride | | 0,15 | | | 0,1 | 0,2 |
| Sodium Benzoate | 0,45 | 0,6 | 0,40 | 0,35 | 0,50 | 0,4 |
| Sodium Salicylate | | | 0,10 | 0,15 | | 0,1 |
| PEG-3 Distearate | | | | 1,0 | | 1,5 |
| Styrene / Acrylates Copolymer | 0,5 | | | | | |
| Zinc Pyrithione | | 0,1 | | | 0,2 | |
| Sodium Chloride | | 0,8 | | | | 1,0 |
| Citric Acid | q.s. | q.s. | q.s. | q.s. | q.s. | q.s. |
| Sodium Hydroxide | q.s. | q.s. | q.s. | q.s. | q.s. | q.s. |
| Parfuem | 1,0 | 0,9 | 0,85 | 1,0 | 1,0 | 1,3 |
| Aqua | ad 100 | ad 100 | ad 100 | ad 100 | ad 100 | ad 100 |

| | | | | | | |
|---|---|---|---|---|---|---|
| *** Nicht erfindungsgemäßes Beispiel. | | | | | | |

### Shampoos (Die Mengenangaben sind Aktivgehalte):

| **Beispiel Nr.** | **7***** | **8***** | **9***** | **10***** | **11***** |
|---|---|---|---|---|---|
| Sodium Laureth Sulfate | 7 | 10 | 12 | 0,11 | 0,12 |
| Cocamidopropyl Betaine | 1,3 | 2,5 | 4 | | |
| Cocamide DEA | 2 | 1,5 | 1 | | |
| Lauryl Glucoside | | | | 4,3 | 4,8 |
| Sodium Myreth Sulfate | | | | 2,8 | 3,2 |
| PEG-80 Sorbitane Laurate | | | | 2,6 | 2,3 |
| Disodium PEG-5 Laurylcitrate Sulfosuccinate | | | | 2,1 | 2,3 |
| AMA-X-Polymer | 1,6 | 1,2 | 1,3 | 2,0 | 1,9 |
| PEG-7 Glyceryl Cocoate | 1,0 | | 1,2 | | 0,5 |
| PEG-40 Hydrogenated Castor Oil | 0,6 | 0,6 | 0,8 | 0,7 | 0,65 |
| Benzophenone-4 | 0,05 | 0,05 | 0,05 | 0,04 | 0,05 |
| Dimethicone | 0,7 | 2,2 | | | |
| Polyquaternium-10 | | | 0,2 | | 0,3 |
| Guar ydroxyproypltrimonium Chloride | 0,3 | | | 0,1 | |
| Sodium Benzoate | 0,2 | 0,45 | 0,40 | 0,35 | 0,4 |
| Sodium Salicylate | 0,2 | | 0,10 | 0,15 | |
| PEG-200 Hydrogenated Glyceryl Palmate | | | | 2 | 1 |
| PEG-90 Glyceryl Isostearate+Laureth-2 | | | | | 1 |
| PEG-3 Distearate | 1,5 | | | | |
| Styrene/Acrylates Copolymer | | | | | 0,5 |
| Climbazole | 0,4 | | | | |
| Piroctone Olamine | | 0,45 | | | |
| Zinc Pyrithione | | | | 0,2 | |
| Sodium Chloride | | 1,0 | | | |
| Citric Acid | q.s. | q.s. | q.s. | q.s. | q.s. |
| Sodium Hydroxide | q.s. | q.s. | q.s. | q.s. | q.s. |
| Parfuem | 1,0 | 0,9 | 0,85 | 1,0 | 1,0 |
| Aqua | ad 100 | ad 100 | ad 100 | ad 100 | ad 100 |

| | | | | | |
|---|---|---|---|---|---|
| *** Nicht erfindungsgemäßes Beispiel. | | | | | |

### Facial Cleanser (die Mengenangaben sind Aktivgehalte):

| **Beispiel Nr.** | **1***** | **2***** | **3** | **4***** | **5** | **5a** | **6***** |
|---|---|---|---|---|---|---|---|
| Sodium Laureth Sulfate | 2,0 | 4 | | | | 1,5 | |
| Cocamidopropyl Betaine | | | 3,8 | 6,2 | 4,3 | | |
| Sodium Cocoamphoacetate | | | | | | | 6,0 |
| Sodium Myreth Sulfate | | | 3,1 | 3,5 | 2,6 | | 3,0 |
| Coco Glucoside | 2,5 | 3,8 | | | | | |
| Lauryl Glucoside | | | 1,0 | | 0,9 | | 2,5 |
| Decyl Glucoside | | | | 2,0 | | 0,26 | |
| Sodium Methyl Cocoyl Taurate | | | | | | 0,45 | |
| AMA-X-Polymer | 1,1 | 1,3 | 1,2 | 1,75 | 1,4 | 1,9 | 1,5 |
| PEG-40 Hydrogenated Castor Oil | 0,6 | 0,5 | 0,5 | 0,4 | 0,5 | | 0,8 |
| PEG-7 Glyceryl Cocoate | | | | | | 0,5 | |
| Benzophenone-4 | 0,05 | 0,05 | 0,05 | | | 0,05 | |
| Polyquaternium-10 | 0,2 | | 0,1 | 0,15 | | | 0,2 |
| Glycerin | | | 1,9 | | 1 | 1,5 | |
| Sodium Benzoate | 0,45 | 0,5 | 0,45 | 0,45 | 0,45 | 0,45 | 0,45 |
| Sodium Salicylate | | | 0,40 | 0,40 | 0,40 | 0,35 | 0,4 |
| PEG-200 Hydrogenated Glyceryl Palmate | | | 0,5 | | 0,7 | | 0,5 |
| PEG-90 Glyceryl Isostearate + Laureth 2 | | | | 1 | | | |
| Panthenol | | | 0,1 | | 0,1 | | |
| Vitamin E Acetate | | 0,1 | | | 0,1 | | |
| Cosmospheres®* | | | | | 0,3 | | |
| Unispheres®** | 0,1 | | | | | | 0,2 |
| Polyethylen particle | | 0,2 | | | | | |
| Citric Acid | q.s. | q.s. | q.s. | q.s. | q.s. | q.s. | q.s. |
| Sodium Hydroxide | q.s. | q.s. | q.s. | q.s. | q.s. | q.s. | q.s. |
| Parfuem | 0,5 | 0,5 | 0,4 | 0,3 | 0,4 | 0,1 | 0,5 |
| Aqua | ad 100 | ad 100 | ad 100 | ad 100 | ad 100 | ad100 | ad100 |

| | | | | | | | |
|---|---|---|---|---|---|---|---|
| *** Nicht erfindungsgemäßes Beispiel. | | | | | | | |

### Flüssigseifen (die Mengenangaben sind Aktivgehalte):

| **Beispiel Nr.** | **1** | **2** | **3***** |
|---|---|---|---|
| Sodium Laureth Sulfate | 5 | 7 | 6,5 |
| Cocamidopropyl Betaine | 4,5 | 3,5 | 5,0 |
| AMA-X-Polymer | 1,28 | 1,44 | 1,5 |
| PEG-7 Glyceryl Cocoate | 1,0 | | 0,5 |
| PEG-40 Hydrogenated Castor Oil | 0,3 | 0,5 | 0,2 |
| Glycerin | | 1 | |
| PEG-120 Methylglucose Dioleate | 0,3 | | |
| Sodium Benzoate | 0,45 | 0,45 | 0,40 |
| Sodium Salicylate | 0,40 | 0,40 | 0,10 |
| **Helianthus Annuus Seed Oil** | 0,1 | | |
| Mandelöl | | 0,1 | |
| Styrene/Acrylates Copolymer | 0,4 | | |
| Glycol Distearate | | | 0,6 |
| Cosmospheres®* | | 0,08 | |
| Unispheres®** | 0,1 | | |
| Cl 10316 | | | 0,002 |
| Cl 16035 | 0,001 | | |
| Trisodium EDTA | 0,1 | | 0,1 |
| Citric Acid | q.s. | q.s. | q.s. |
| Sodium Hydroxide | q.s. | q.s. | q.s. |
| Parfuem | 0,5 | 0,2 | 0,4 |
| Aqua | ad 100 | ad 100 | ad 100 |

| | | | |
|---|---|---|---|
| *** Nicht erfindungsgemäßes Beispiel. *Cosmospheres®: INCI-Bezeichnung: Lactose+Microcrystalline Cellulose+Helianthus Annuus Seed Oil+Cl 77492 (optional auch mit anderen Wirk- und Farbstoffen als Helianthus Annus Seed und Cl 77492), als feste Partikel. ** Unispheres®: INCI-Bezeichnung: Lactose+Cellulose+Cl 77007+Hydroxypropyl Methylcellulose+Tocopheryl Acetate (optional auch mit anderen Wirk- und Farbstoffen als Tocopheryl Acetat und Cl 77007), als feste Partikel. | | | |

### Vergleichsversuch zur Bestimmung der Milde:

| ***Erfindungsgemäße Zubereitung:*** | | |
|---|---|---|
| ***Polymerphase*** | [g] | |
| Aqua | 343,7 | vorlegen |
| AMA-X-Polymer (ca. 32%ig) | 49,4 | zugeben |
| | | |

| ***Tensidphase*** | | |
|---|---|---|
| Sodium Laureth Sulfate (25% ig) | 360,0 | Zur Polymerphase geben |
| | | |

| ***Tensidphase*** | | |
|---|---|---|
| [Cocamidopropyl Betain + Glycerin] (34%ig) | 89,0 | Zur Tensid-/Polymerphase geben |

| ***NaOH - phase*** | | pH-Wert der Tensid-/Polymerphase auf 5,9 einstellen! |
|---|---|---|
| Sodium Hydroxide (45%ig) | ca. 3,0 | |
| | | |

| ***Lichtfilterphase*** | | Benzophenone-4 in Wasser lösen; Trisodium EDTA zugeben; Zur Tensid-/Polymerphase geben |
|---|---|---|
| Aqua | 30,0 | |
| Benzophenone-4 | 0,5 | |
| Trisodium EDTA (20%ig) | 10,0 | |
| | | |

| ***Parfümphase*** | | Bis 40°C auf dem Magnetrührer lösen; Zur Tensid-/Polymerphase geben |
|---|---|---|
| PEG-40 Hydrogenated Castor Oil | 6,0 | |
| Helianthus Annuus Seed Oil | 0,1 | |
| PEG-7 Glyceryl Cocoate | 10,0 | |
| Parfum | 13,0 | |
| | | |

| ***Konservierungsmittelphase*** | | Sodium Benzoate in Wasser lösen; Zur Tensid-/Polymerphase geben |
|---|---|---|
| Aqua | 30,0 | |
| Sodium Benzoate | 4,5 | |

| ***Citronensäure*** | | pH-Wert auf 4,8 - 5,2 einstellen |
|---|---|---|
| Aqua | 4,5 | |
| Citric Acid | 0,5 | |
| | | |

| ***Beads*** | | Zum Ansatz geben |
|---|---|---|
| Lactose+Microcrystalline Cellulose+Helianthus Annuus Seed Oil+Cl77492 | 0,8 | |
| **Ansatzmenge gesamt** | **1000,0** | |

Mit der so erhaltenen erfindungsgemäßen Zubereitung und einem Handelsprodukt (Balea Dusche & Ölperlen Queen of the Night) wurde ein Standard-RBC-Test (WAS) zur Bestimmung der Milde durchgeführt.

| Prüfmuster | H⁵⁰ [µl/ml] | DI | L/D |
|---|---|---|---|
| Balea Dusche&Ölperlen Queen oft he Night | 16,6 | 50,3 | 0,33 |
| Erfindungsgemäße Zubereitung | 16,5 | 34,0 | 0,48 |

Die L/D-Werte, die eine Aussage über die Milde einer Zubereitung erlauben, sind für die erfindungsgemäße Zubereitung höher als für das Handelsprodukt. Dies zeigt, dass die Milde der erfindungsgemäßen Zubereitung besser ist.

## Patentansprüche

1. Wässrige, tensidhaltige Zubereitungen,
enthaltend ein oder mehrere vernetzte Acrylat Copolymere,
wobei die Acrylat Copolymere AMA-X-Polymere sind,
erhältlich durch radikalische Emulsionspolymerisation von
(A) wenigstens einem sauren Vinylmonomer oder dessen Salz, wobei das saure Vinylmonomer oder dessen Salz (A) gewählt wird unter Acrylsäure oder Methacrylsäure oder deren Alkali-, Erdalkali-, Ammonium oder Alkylammoniumsalzen,
(B) wenigstens einem nichtionischen Vinylmonomer, wobei das nichtionische Vinylmonomer (B) gewählt wird aus der Gruppe der C1-C22-Alkylacrylate und der C1-C22 Alkylmethacrylate sowie deren Mischungen,
(C) wenigstens einem Monomer, enthaltend eine ungesättigte Endgruppe und einen Polyoxyalkylenteil, wobei das Monomer (C) gewählt wird unter Allylpolyethylenglykolether mit 30 Ethylenoxyeinheiten, Allylpolyethylenglykolether mit 20 Ethylenoxyeinheiten, Vinylpolyethylenglykolether mit 20 Ethylenoxyeinheiten und CH2=CHCH2O[(CH2CH2O)n(CH2(CH3)CHO)]mCH3 mit m+n = 5 bis 100 und n/m = 1,
(D) wenigstens einem quervernetzenden Monomeren, wobei das quervernetzende Monomer (D) gewählt wird unter Polyol(meth)acrylaten mit wenigstens zwei (Meth)acrylatgruppen und den Mischestern von Polyolen mit Acrylsäure und/oder Methacrylsäure,
(E) optional einem Schutzkolloid
**dadurch gekennzeichnet, dass** die Polymerisation so gesteuert wird, dass (F) zumindest zeitweise der Geleffekt auftritt, dadurch erreicht, dass die Monomerzugabe der Monomere (A), (B) und (C) (Dosierzeit) während 120 Minuten, bevorzugt 60 Minuten, besonders bevorzugt 40 Minuten, ganz besonders bevorzugt 30 Minuten erfolgt und (G) die Zugabe des quervernetzenden Monomeren (D) frühestens 10 Minuten, bevorzugt frühestens 15 Minuten nach der ersten Zugabe der Monomere (A), (B) und (C) beginnt,
wobei (H) assoziative Monomere fehlen oder höchstens eine Konzentration von 1 Gew.%, bevorzugt 0,1 Gew.% aufweisen,
anionische Tenside mit einem Gehalt von 1,0 bis < 12 Gew.-%, bezogen auf das Gesamtgewicht der Zubereitung und
Haut- und/oder Haarpflegesubstanzen, ausgewählt aus Glycerin, pflanzlichen Ölen, Paraffinölen oder Panthenol, einzeln oder Kombination.

2. Zubereitungen nach Anspruch 1, **dadurch gekennzeichnet, dass** sie einen Gehalt von 0,1 bis 5,0 %, bevorzugt 0,5 bis 4,0 %, besonders bevorzugt 1,0 bis 2,0 %, bezogen auf den Aktivgehalt und das Gesamtgewicht der Zubereitung, an AMA-X-Polymeren enthalten.

3. Zubereitung nach Anspruch 1 und/oder 2, **dadurch gekennzeichnet, dass** Partikel, Tröpfchen und/oder Blasen nahezu homogen in den Zubereitungen verteilt sind und bleiben.

4. Zubereitungen nach wenigstens einem der vorstehenden Ansprüche, **dadurch gekennzeichnet, dass** sie einen pH-Wert von 4 bis 7, bevorzugt 4,0 bis < 6,4, besonders bevorzugt 4,0 bis ≤ 5,5 aufweisen.

5. Zubereitungen nach wenigstens einem der vorstehenden Ansprüche, **dadurch gekennzeichnet, dass** neben AMA-X-Polymeren zusätzlich weitere Struktur-bildende Polymere enthalten sind.

6. Zubereitungen nach wenigstens einem der vorstehenden Ansprüche, **dadurch gekennzeichnet, dass** der Gehalt an AMA-X-Polymeren und weiteren Struktur-bildenden Polymeren 0,1 bis 5,0 %, bevorzugt 0,2 bis 4,0 %, besonders bevorzugt 0,5 bis 2,0 % beträgt, bezogen auf den Aktivgehalt.

7. Zubereitungen nach mindestens einem der vorstehenden Ansprüche, **dadurch gekennzeichnet, dass** sie transparent sind.

8. Zubereitungen nach Patentanspruch 8, **dadurch gekennzeichnet, dass** die Trübungswerte unter 30 NTU, bevorzugt unter 25 NTU, besonders bevorzugt unter oder gleich 20 NTU liegen.

9. Zubereitungen nach mindestens einem der vorstehenden Ansprüche, **dadurch gekennzeichnet, dass** die Haut- und/oder Haarpflegesubstanzen gemäß Anspruch 1 mit einem Gehalt an 0,01 bis 10 Gew.-%, bevorzugt 0,1 bis 8 Gew.-%, bezogen auf das Gesamtgewicht der Zubereitung, enthalten sind.

10. Zubereitungen nach mindestens einem der vorstehenden Ansprüche, **dadurch gekennzeichnet, dass** sie 0,1 bis 3,0 Gew.-% eines oder mehrerer anorganischen/r Salze(s), ausgewählt aus der Gruppe der Alkalimetallhalogenide, der Alkalimetallsulfate, der Alkalimetallnitrate und der Alkalimetallphosphate, enthalten.

## Claims

1. Aqueous, surfactant-containing preparations
comprising one or more crosslinked acrylate copolymers,
where the acrylate copolymers are AMA-X polymers
obtainable by free-radical emulsion polymerization of
(A)at least one acidic vinyl monomer or salt thereof, the acidic vinyl monomer or salt thereof (A) being selected from acrylic acid or methacrylic acid or alkali metal, alkaline earth metal, ammonium or alkylammonium salts thereof,
(B)at least one nonionic vinyl monomer, the nonionic vinyl monomer (B) being selected from the group of C1-C22 alkyl acrylates and C1-C22 alkyl methacrylates and also mixtures thereof,
(C)at least one monomer comprising an unsaturated end group and a polyoxyalkylene moiety, the monomer (C) being selected from allylpolyethylene glycol ethers having 30 ethyleneoxy units, allylpolyethylene glycol ethers having 20 ethyleneoxy units, vinylpolyethylene glycol ethers having 20 ethyleneoxy units, and CH2=CHCH2O[(CH2CH2O)n(CH2(CH3)CHO)]mCH3 where m+n = 5 to 100 and n/m = 1,
(D)at least one crosslinking monomer, the crosslinking monomer (D) being selected from polyol(meth)acrylates with at least two (meth)acrylate groups and the mixed esters of polyols with acrylic acid and/or methacrylic acid,
(E)optionally a protective colloid,
**characterized in that** the polymerization is controlled such that (F) the gel effect occurs at least temporarily, achieved **in that** the monomer addition of the monomers (A), (B) and (C) (metering period) takes place over 120 minutes, preferably 60 minutes, particularly preferably 40 minutes, especially preferably 30 minutes, and (G) the addition of the crosslinking monomer (D) begins at the earliest 10 minutes, preferably at the earliest 15 minutes, after the first addition of the monomers (A), (B) and (C),
where (H) associative monomers are absent or at most have a concentration of 1% by weight, preferably 0.1% by weight,
anionic surfactants at a content of 1.0 to < 12% by weight, based on the total weight of the preparation, and
skincare and/or haircare substances selected from glycerol, vegetable oils, paraffin oils or panthenol, individually or in combination.

2. Preparations according to Claim 1, **characterized in that** they comprise a content of 0.1 to 5.0%, preferably 0.5 to 4.0%, particularly preferably 1.0 to 2.0% of AMA-X polymers, based on the active content and the total weight of the preparation.

3. Preparation according to Claim 1 and/or 2, **characterized in that** particles, droplets and/or bubbles are virtually homogeneously distributed and remain in the preparations.

4. Preparations according to at least one of the preceding claims, **characterized in that** they have a pH of 4 to 7, preferably 4.0 to < 6.4, particularly preferably 4.0 to ≤ 5.5.

5. Preparations according to at least one of the preceding claims, **characterized in that** additionally further structure-forming polymers are present in addition to AMA-X polymers.

6. Preparations according to at least one of the preceding claims, **characterized in that** the content of AMA-X polymers and further structure-forming polymers is 0.1 to 5.0%, preferably 0.2 to 4.0%, particularly preferably 0.5 to 2.0%, based on the active content.

7. Preparations according to at least one of the preceding claims, **characterized in that** they are transparent.

8. Preparations according to Claim 8, **characterized in that** the turbidity values are below 30 NTU, preferably below 25 NTU, particularly preferably below or equal to 20 NTU.

9. Preparations according to at least one of the preceding claims, **characterized in that** the skincare and/or haircare substances according to Claim 1 are present at a content of 0.01 to 10% by weight, preferably 0.1 to 8% by weight, based on the total weight of the preparation.

10. Preparations according to at least one of the preceding claims, **characterized in that** they comprise 0.1 to 3.0% by weight of one or more inorganic salt(s) selected from the group of the alkali metal halides, the alkali metal sulfates, the alkali metal nitrates and the alkali metal phosphates.

## Revendications

1. Compositions aqueuses, contenant des tensioactifs, contenant un ou plusieurs copolymères réticulés d'acrylate, les copolymères d'acrylate étant des polymères de type AMA-X pouvant être obtenus par polymérisation radicalaire en émulsion de
(A) au moins un monomère acide de vinyle ou son sel, le monomère acide de vinyle ou son sel (A) étant choisi parmi l'acide acrylique ou l'acide méthacrylique ou leurs sels de métal alcalin, alcalino-terreux, d'ammonium ou d'alkylammonium,
(B) au moins un monomère non ionique de vinyle, le monomère non ionique de vinyle (B) étant choisi dans le groupe des acrylates d'alkyle en C1-C22 et des méthacrylates d'alkyle en C1-C22 ainsi que de leurs mélanges,
(C) au moins un monomère contenant un groupe terminal insaturé et une proportion de polyoxyalkylène, le monomère (C) étant choisi parmi les allylpolyéthylèneglycoléthers comprenant 30 unités d'oxyde d'éthylène, les allylpolyéthylèneglycoléthers comprenant 20 unités d'oxyde d'éthylène, les vinylpolyéthylèneglycoléthers comprenant 20 unités d'oxyde d'éthylène et CH₂=CHCH₂O[(CH₂CH₂O)ₙ(CH₂(CH₃)CHO)]ₘCH₃ où m + n = 5 à 100 et n/m = 1,
(D) au moins un monomère réticulant, le monomère réticulant (D) étant choisi parmi les (méth)acrylates de polyol comprenant au moins deux groupes (méth)acrylate et les esters mixtes de polyols avec de l'acide acrylique et/ou de l'acide méthacrylique,
(E) éventuellement un colloïde de protection
**caractérisées en ce que** la polymérisation est régulée de manière telle que (F) au moins un effet de gel se produit temporairement, obtenu **en ce que** l'addition des monomères (A), (B) et (C) (temps de dosage) a lieu pendant 120 minutes, de préférence pendant 60 minutes, de manière particulièrement préférée pendant 40 minutes, de manière tout particulièrement préférée pendant 30 minutes et (G) l'addition du monomère réticulant (D) commence au plus tôt 10 minutes, de préférence au plus tôt 15 minutes après la première addition des monomères (A), (B) et (C),
(H) des monomères associatifs étant absents ou présentant une concentration d'au plus 1% en poids, de préférence de 0,1% en poids,
des tensioactifs anioniques présentant une teneur de 1,0 à < 12% en poids, par rapport au poids total de la composition et
des substances de soin de la peau et/ou des cheveux, choisies parmi le glycérol, les huiles végétales, les huiles de paraffine ou le panthénol, seules ou en combinaison.

2. Compositions selon la revendication 1, **caractérisées en ce qu'**elles contiennent une teneur de 0,1 à 5,0%, de préférence de 0,5 à 4,0%, de manière particulièrement préférée de 1,0 à 2,0%, par rapport à la teneur active et au poids total de la composition, de polymères de type AMA-X.

3. Composition selon la revendication 1 et/ou 2, **caractérisée en ce que** des particules, des gouttelettes et/ou des bulles sont réparties et restent de manière quasiment homogène dans les compositions.

4. Compositions selon au moins l'une quelconque des revendications précédentes, **caractérisées en ce qu'**elles présentent un pH de 4 à 7, de préférence de 4,0 à < 6,4, de manière particulièrement préférée de 4,0 à ≤ 5,5.

5. Compositions selon au moins l'une quelconque des revendications précédentes, **caractérisées en ce qu'**elles contiennent en outre, en plus des polymères de type AMA-X, d'autres polymères structurants.

6. Compositions selon au moins l'une quelconque des revendications précédentes, **caractérisées en ce que** la teneur en polymères de type AMA-X et en autres polymères structurants est de 0,1 à 5,0%, de préférence de 0,2 à 4,0%, de manière particulièrement préférée de 0,5 à 2,0%, par rapport à la teneur active.

7. Compositions selon au moins l'une quelconque des revendications précédentes, **caractérisées en ce qu'**elles sont transparentes.

8. Compositions selon la revendication 8, **caractérisées en ce que** la valeur de trouble est inférieure à 30 NTU, de préférence inférieure à 25 NTU, de manière particulièrement préférée inférieure ou égale à 20 NTU.

9. Compositions selon au moins l'une quelconque des revendications précédentes, **caractérisées en ce que** les substances de soin de la peau et/ou des cheveux selon la revendication 1 sont contenues en une teneur de 0,01 à 10% en poids, de préférence de 0,1 à 8% en poids, par rapport au poids total de la composition.

10. Compositions selon au moins l'une quelconque des revendications précédentes, **caractérisées en ce qu'**elles contiennent 0,1 à 3,0% en poids d'un ou de plusieurs sels inorganiques, choisis dans le groupe des halogénures de métal alcalin, des sulfates de métal alcalin, des nitrates de métal alcalin et des phosphates de métal alcalin.
